# EUROPÄISCHE PATENTSCHRIFT

(11) **EP 2 281 500 B1**
(45) Veröffentlichungstag und Bekanntmachung des Hinweises auf die Patenterteilung: **08.02.2012**
(21) Anmeldenummer: 09010118.9
(22) Anmeldetag: 05.08.2009
(51) Int. Cl.: A61B 3/10, A61B 3/117

(54) **Ophthalmologische Messvorichtung und Messverfahren**
Ophthalmologic measuring device and measuring method
Dispositif de mesure ophtalmologique et son procédé de mesure

(43) Veröffentlichungstag der Anmeldung: 09.02.2011
(73) Patentinhaber: SIS AG, Surgical Instrument Systems, 2562 Port (CH)
(72) Erfinder: Rathjen, Christian, 28197 Bremen (DE)
(74) Vertreter: Vogel, Dany

(56) Entgegenhaltungen:
- EP-A- 1 430 829
- EP-A- 2 020 205
- WO-A-03/101355
- DE-A1-102007 017 599
- US-A- 5 886 768

## Beschreibung

### Technisches Gebiet

Die vorliegende Erfindung betrifft eine ophthalmologische Messvorrichtung und ein ophthalmologisches Messverfahren zum Bestimmen von geometrischen Strukturen eines Auges. Die vorliegende Erfindung betrifft insbesondere eine ophthalmologische Messvorrichtung und ein ophthalmologisches Messverfahren zum Bestimmen von geometrischen Strukturen eines Auges, in welchen ein optisches, triangulierendes erstes Messsystem und ein optisches, interferometrisches zweites Messsystem verwendet werden.

### Stand der Technik

Die Offenlegungsschrift DE 10 2007 017 599 beschreibt ein ophthalmologisches Messsystem und ein ophthalmologisches Messverfahren zum Bestimmen von geometrischen Parametern im vorderen Augenabschnitt, die zur Berechnung der Brechkraft von Intraokularlinsen erforderlich sind. Das Messsystem nach DE 10 2007 017 599 umfasst kombiniert in einem einzigen Gerät eine erste Messvorrichtung zur Bestimmung der Achslänge sowie eine zweite Messvorrichtung zum Erfassen mehrerer Strukturen im Vorderabschnitt, wie Hornhaut, Vorderkammer und Linse. Dabei umfasst die erste Messvorrichtung ein bekanntes im Zeit- oder Frequenzraum arbeitendes optisches Kohärenzinterferometer (PCI = Partial Coherence Interferometry, OCT = Optical Coherence Tomography). Die zweite Messvorrichtung umfasst eine Spaltbeleuchtungseinheit und eine Bildaufzeichnungseinheit zur Ausführung eines bekannten Schnittbildverfahrens. Das Messsystem umfasst zudem eine Steuereinheit, welche eingerichtet ist, sowohl die für die Achslängenmessung erforderlichen Messwerte als auch die für eine Triangulation am Auge erforderlichen Winkel- und Abstandsverhältnisse auszuwerten, wobei aus den von der Bildaufzeichnungseinheit gelieferten Schnittbildern der vorderen Augenabschnitte Teilstrecken, Radien und/oder Brechungswinkel bestimmt werden.

Der Vorteil von triangulierenden Messvorrichtungen mit einem Schnittbildverfahren gegenüber solchen die auf einem oder mehreren Einzelstrahlen basieren besteht darin, dass triangulierende Schnittbildverfahren keine oder nur wenige durch Augenbewegungen verursachte Bewegungsartefakte erzeugen und somit eine vergleichsweise höhere geometrische Präzision bei gleicher Anzahl von Messpunkten aufweisen. In Scheimpflugkonfiguration besteht zudem der weitere Vorteil, dass der Tiefenmessbereich bei hoher optischer Auflösung wesentlich grösser ausgelegt werden kann, als bei interferometrischen Verfahren. Dies gilt sowohl für die laterale Auflösung als auch für die Tiefenauflösung. Bei Triangulationsverfahren wie auch bei interferometrischen Verfahren mit einem einzelnen gescannten Strahl erzeugen die durch Augenbewegungen verursachten Bewegungsartefakte Messabweichungen, die über ein zusätzliches, die Augenbewegungen registrierendes Verfahren korrigiert werden müssen. Ein solches, bis anhin nicht bekanntes Verfahren müsste, um wirksam zu sein, wesentlich schneller und präziser sein, als die durch die Augenbewegungen verursachten Messabweichungen. Triangulationsverfahren mit vielen Einzelstrahlen bzw. mit strukturierter Beleuchtung weisen ihrerseits den Nachteil auf, dass die Anzahl der Messpunkte begrenzt und der Tiefenmessbereich überdies stark eingeschränkt ist, da die Bildaufzeichnungseinheit nicht für sämtliche Strahlen in Scheimpflugkonfiguration angeordnet werden kann. Die bekannten triangulierenden Messvorrichtungen weisen allgemein den Nachteil auf, dass ihr erfassbarer Messbereich auf den direkt ausleuchtbaren vorderen Augenbereich (Vorderkammerbereich) beschränkt ist, und die hintere Augenkammer nicht einsehbar ist. Dabei wird der Messbereich der bekannten triangulierenden Messvorrichtungen durch Trübungen, zB. in der Hornhaut, Implantate, z.B. vorgesetzte Linsen, und durch die Iris weiter eingeschränkt, und gewisse Teilbereiche, wie der Vorderkammerwinkel, sind auf Grund von Gewebeeigenschaften für die bekannten triangulierenden Messvorrichtungen ganz allgemein nicht erfassbar.

Der Vorteil von bekannten optischen Kohärenzinterferometern besteht darin, dass ihr erfassbarer Messbereich nicht auf den vorderen Augenbereich (Vorderkammerbereich) beschränkt ist, da aufgrund ihres axialen Arbeitsprinzips auch die hintere Augenkammer ausmessbar ist. Bei herkömmlichen interferometrischen Verfahren wird typischerweise ein scannender Strahl verwendet Dabei erzeugt der Strahl an einer bestimmten Position einen Tiefenscan (A-Scan) an dieser Stelle. Durch relatives Verschieben von Messobjekt und Lichtstrahl, respektive Scannen des Strahls, wird der Tiefenscan über eine zusammenhängende Fläche entlang einer Scanlinie ausgeweitet (B-Scan). Ein dreidimensionaler Scan (C-Scan) wird aus mehren B-Scans zusammengesetzt. Sobald die interferometrischen Verfahren daher zur strukturellen Messung an mehreren Positionen eingesetzt werden, werden durch Augenbewegungen Bewegungsartefakte und somit Messabweichungen erzeugt Überdies besteht gegenüber triangulierenden Messverfahren in Scheimpflugkonfiguration der weitere Nachteil, dass der Tiefenmessbereich von interferometrischen Verfahren bei hoher lateraler Auflösung wesentlich kleiner ausgelegt werden muss. Da aus optischen Gründen der Tiefenschärfebereich überproportional mit der Bildauflösung abnimmt, schränkt der Wunsch nach hoher lateraler Auflösung den Tiefenmessbereich allgemein und insbesondere bei interferometrischen Verfahren signifikant ein.

Es sei hier zudem erwähnt, dass es weitere interferometrische (OCT-) Verfahren gibt, die über optische Parallelisierung mehrere AScans (mit mehreren Lichtstrahlen) gleichzeitig erfassen können. Weiterhin gibt es En-Face-Techniken, die nacheinander mehrere Ebenen in der Tiefe scannen um dann anschliessend A- und B-Scans zu synthetisieren. Einige dieser Techniken können dazu eingesetzt werden, Bewegungsartefakte zu reduzieren. Nachteilig sind jedoch der dafür erforderliche hohe technische und finanzielle Aufwand, eine reduzierte Auflösung und/oder Kompromisse (Einschränkungen) beim Tiefenmessbereich. Es kann festgestellt werden, dass keine kommerziell verfügbaren Systeme mit bewegungs- und artefaktfreien interferometrischen Verfahren existieren. Einen guten Überblick über den Stand der Technik wird in [Drexler] wieder gegeben.

Aus der Patentameldung EP-A-2 020 205 der SIS AG, Surgical Instrument Systems, ist eine Messvorrichtung mit den Merkmalen des ersten Teils von Patentanspruch 1 bekannt.

Auch wenn triangulierende Verfahren und interferometrische (OCT-) Verfahren für die geometrische Messtechnik verwendet werden können, sei hier festgehalten, dass sie auf anderen Wirkprinzipien beruhen. Während das Funktionsprinzip von triangulierenden Verfahren auf diffuser Streuung im Gewebe und an Oberflächen in einem grossen Raumbereich basiert, arbeiten interferometrische Verfahren in Rückwärtsreflexion zur projizierenden Optik. Damit liefern beide Verfahren neben der Geometrie unterschiedliche und damit insgesamt mehr Informationen über die strukturelle Beschaffenheit des Messobjekts.

Es ist eine Aufgabe der vorliegenden Erfindung eine ophthalmologische Messvorrichtung und ein ophthalmologisches Messverfahren zum Bestimmen von geometrischen Strukturen eines Auges vorzuschlagen, welche zumindest einige Nachteile der bekannten Systeme nicht aufweisen. Es ist insbesondere eine Aufgabe der vorliegenden Erfindung eine ophthalmologische Messvorrichtung und ein ophthalmologisches Messverfahren zum Bestimmen von geometrischen Strukturen eines Auges vorzuschlagen, welche einerseits nicht auf den einsehbaren Messbereich von bekannten triangulierenden Messverfahren beschränkt sind und andererseits eine verminderte Beeinträchtigung der Messgenauigkeit durch Bewegungsartefakte gegenüber bekannten interferometrischen Verfahren aufweisen.

Gemäss der vorliegenden Erfindung werden diese Ziele insbesondere durch die Elemente der unabhängigen Ansprüche erreicht. Weitere vorteilhafte Ausführungsformen gehen ausserdem aus den abhängigen Ansprüchen und der Beschreibung hervor.

Die oben genannten Ziele werden durch die vorliegende Erfindung insbesondere dadurch erreicht, dass eine ophthalmologische Messvorrichtung zum Bestimmen von geometrischen Strukturen eines Auges, insbesondere geometrische Strukturen im Vorderkammerbereich des Auges, ein optisches, triangulierendes erstes Messsystem zur Bestimmung von mindestens einer geometrischen Referenz im Auge durch Triangulation, und ein optisches, interferometrisches zweites Messsystem zur Bestimmung von (zwei- und/oder dreidimensionalen) geometrischen Detailstrukturen im Auge (insbesondere im Vorderkammerbereich des Auges) durch optische Interferometrie umfasst, und dass die Messvorrichtung eingerichtet ist, die durch das zweite Messsystem bestimmten geometrischen Detailstrukturen basierend auf der durch das erste Messsystem bestimmten mindestens einen geometrischen Referenz im Auge zu positionieren. Da triangulierende Messsysteme, insbesondere Lichtspalt- (Lichtschnitt-) basierte Messsysteme, keine oder nur geringfügige Bewegungsartefakte erzeugen (es genügen bei triangulierenden Messsystemen beispielsweise 30 bis 60 Lichtspaltaufnahmen für eine Gesamttopologie der Cornea, wohingegen bei interferometrischer Vermessung dafür über 500 axiale Messungen in 30 bis 60 Scanflächen erforderlich wären), und da interferometrische Messsysteme für einen im Vergleich zum Messbereich des triangulierenden Messsystems kleineren Detailmessbereich mit hoher optischer Auflösung ausgelegt werden können, ermöglicht die Positionierung der geometrischen Detailstrukturen im Auge basierend auf der geometrischen Referenz eine besonders vorteilhafte Kombination von triangulierenden und interferometrischen Messverfahren. Geometrische Detailstrukturen können einerseits durch das interferometrische Messsystem im Auge mit hoher optischer Auflösung und an für das triangulierende Messsystem nicht einsehbaren Stellen erfasst werden, und andererseits im Auge basierend auf geometrischen Referenzen positioniert werden, welche durch das triangulierende Messsystems ohne signifikante Bewegungsartefakte bestimmt werden. Somit werden die Vorteile des interferometrischen Messsystems mit den Vorteilen des triangulierenden Messsystems kombiniert. Die verminderte Beeinträchtigung gegenüber Bewegungsartefakten ermöglicht zudem dem Einsatz von kostengünstigen, langsameren interferometrischen (OCT-) Techniken (insbesondere Time Domain OCT), die vorteilhaft grössere Tiefenmessbereiche besitzen und auch die Verwendung anderer Wellenlängen ermöglichen (insbesondere Wellenlängen > 1000 nm).

In einer Ausführungsvariante sind das erste Messsystem und das zweite Messsystem mechanisch so gekoppelt, dass die geometrischen Detailstrukturen im Auge bei einer der geometrischen Referenz entsprechenden Stellung des ersten Messsystems (11) basierend auf einem oder mehreren geometrischen Einstellwerten positionierbar sind. Mit anderen Worten, das triangulierende Messsystem und das interferometrische Messsystem werden mechanisch/geometrisch, vorzugsweise über einen Kalibrierkörper, für die Erfassung einer oder mehrerer definierten geometrischen Referenzen des Auges eingestellt und aus dieser definierten geometrischen Kalibrierstellung werden die geometrischen Einstellwerte bestimmt, welche eine kalibrierte Beziehung zwischen dem triangulierenden Messsystem und dem interferometrischen Messsystem definieren und eine Positionierung der durch das interferometrische Messsystem bestimmten geometrischen Detailstrukturen bei einer definierten Stellung des triangulierenden Messsystems (in Bezug zum Auge) ermöglichen. Diese Variante hat den Vorteil, dass keine bildtechnische Erfassung und Zuordnung von geometrischen Strukturmerkmalen und eine darauf basierte Positionierung der geometrischen Detailstrukturen erforderlich ist, wenn die Erfassung der geometrischen Detailstrukturen durch das interferometrische Messsystem zeitnah (d.h. innerhalb eines definierten Zeitfensters) zu der der geometrischen Referenz entsprechenden Stellung des triangulierenden Messsystems erfolgt.

In einer weiteren Ausführungsvariante umfasst die Messvorrichtung ein Positionierungsmodul, welches eingerichtet ist, eines oder mehrere geometrische Referenzmerkmale des Auges zu bestimmen, und die geometrischen Detailstrukturen basierend auf den geometrischen Referenzmerkmalen im Auge zu positionieren. Mit anderen Worten, die geometrischen Detailstrukturen werden im Auge auf der Basis von geometrischen Referenzmerkmalen des Auges positioniert, welche einerseits als geometrische Referenz durch das triangulierende Messsystem und andererseits in den geometrischen Detailstrukturen durch das interferometrische Messsystem bestimmt werden. Diese Variante hat den Vorteil, dass ein Benutzer keine mechanische/geometrische Einstellung des triangulierenden Messsystems auf eine definierte geometrische Referenz des Auges vornehmen muss, bevor die geometrischen Detailstrukturen durch das das interferometrische Messsystem erfassen kann. Das heisst das triangulierende und das interferometrische Messverfahren können ohne störende Einflüsse von Augenbewegungen (zeitlich) unabhängig voneinander ausgeführt werden, also sowohl parallel/zeitgleich als auch sequentiell und zeitlich verschoben.

Vorzugsweise umfasst das erste Messsystem einen Lichtprojektor zur Projektion eines Lichtspalts (Lichtschnitt) und ein erstes Scanmodul zum Bewegen des Lichtspalts an verschiedene Stellen des Auges, und das zweite Messsystem umfasst ein Interferometer und ein zweites Scanmodul, wobei das zweite Scanmodul eingerichtet ist, an mehreren unterschiedlichen Positionen des Auges mindestens einen Lichtstrahl vom Interferometer auf und ins Auge zu projizieren und vom Auge reflektiertes Licht dem Interferometer zuzuführen (parallel arbeitende Interferometer verwenden gleichzeitig mehrere Lichtstrahlen).

In einer Variante weisen das triangulierende erste Messsystem und das interferometrische zweite Messsystem eine gemeinsame Lichtquelle auf. Insbesondere liefert das Interferometer nicht nur das Licht für den Lichtstrahl, sondern auch das Licht für den Lichtspalt (Lichtschnitt) des triangulierenden Messsystems.

In einer Ausführungsvariante ist das erste Scanmodul eingerichtet, den Lichtspalt entlang einer Scanrichtung zu bewegen, und das zweite Scanmodul ist eingerichtet, den Lichtstrahl in einer zur Scanrichtung im Wesentlichen parallel oder im Wesentlichen normal verlaufenden Scanebene zu bewegen. Im ersteren Fall verläuft die Abtastung durch das interferometrische Messsystem beispielsweise senkrecht zum Lichtspalt, im letzteren Fall beispielsweise im Lichtspalt oder parallel dazu. Die Bewegung des Lichtspalt und des Lichtstrahls erfolgt jeweils durch Ablenkung mittels eines Ablenkspiegels oder durch Verschiebung eines entsprechenden Projektors oder Spiegels mittels eines Bewegungstreibers.

In einer weiteren Ausführungsvariante ist das erste Scanmodul eingerichtet, den Lichtspalt durch Rotation um eine im Wesentlichen im Lichtspalt verlaufende Drehachse zu bewegen, und das zweite Scanmodul ist eingerichtet, den Lichtstrahl in einer Scanebene zu bewegen, wobei eine zentrale Projektionsachse des zweiten Scanmoduls mit der Drehachse einen Winkel bildet oder entlang der Drehachse verläuft. Ein Abtasten des Auges durch eine Rotation des Lichtspalts erlaubt eine besonders einfache Ausführungsform des triangulierenden Messsystems, ohne Ablenkspiegel oder translatorische Bewegungstreiber.

In einer anderen Ausführungsvariante sind das erste Scanmodul und das zweite Scanmodul so eingerichtet und gekoppelt, dass der Lichtspalt und die Scanebene in einer gemeinsamen Ebene liegen.

In einer weiteren Ausführungsvariante weisen der vom Lichtprojektor projizierte Lichtspalt und der Lichtstrahl vom Interferometer unterschiedliche Wellenlängen auf. Zum Beispiel beträgt die Wellenlänge des projizierten Lichtspalts weniger als 500nm und die Wellenlänge des Lichtstrahls vom Interferometer mehr als 1000nm insbesondere 1300nm.

Vorzugsweise umfasst das erste Messsystem mindestens eine in Scheimpflug angeordnete Kamera zum Erfassen von durch den Lichtspalt beleuchteten Querschnittsteilen des Auges.

In einer weiteren Ausführungsvariante umfasst die Messvorrichtung ein Verarbeitungsmodul, welches eingerichtet ist, mehrere im Auge verschieden positionierte geometrische Detailstrukturen zu erfassen, diese geometrischen Detailstrukturen basierend auf der mindestens einen geometrischen Referenz gegenseitig zu positionieren und zu einer übergeordneten kombinierten geometrischen Detailstruktur zusammenzusetzen.

In einer Ausführungsvariante ist das Verarbeitungsmodul eingerichtet, die geometrischen Detailstrukturen zum Zusammensetzen der übergeordneten kombinierten geometrischen Detailstruktur passend zu verformen.

Vorzugsweise ist das erste Messsystem eingerichtet, die geometrische Referenz in einem definierten Referenzmessbereich des Auges zu bestimmen, das zweite Messsystem ist eingerichtet, die geometrischen Detailstrukturen in einem im Verhältnis zum definierten Referenzmessbereich wesentlich kleineren Detailmessbereich des Auges zu bestimmen, und die Messvorrichtung umfasst Bewegungstreiber zum Bewegen des zweiten Messsystems zum Bestimmen von geometrischen Detailstrukturen in mehreren verschiedenen Detailmessbereichen des Auges.

In einer Ausführungsvariante kann der Detailmessbereich des Auges innerhalb des definierten Referenzmessbereichs, überlappend mit dem definierten Referenzmessbereich, angrenzend an den definierten Referenzmessbereich und/oder ausserhalb des definierten Referenzmessbereichs angeordnet werden.

Die vorliegende Erfindung bezieht sich zudem auf ein ophthalmologisches Messverfahren zum Bestimmen von geometrischen Strukturen eines Auges, welches folgende Schritte umfasst: Bestimmen von mindestens einer geometrischen Referenz im Auge durch ein optisches, triangulierendes erstes Messsystem, Bestimmen von geometrischen Detailstrukturen im Auge durch ein optisches interferometrisches zweites Messsystem, und Positionieren der durch das zweite Messsystem bestimmten geometrischen Detailstrukturen im Auge basierend auf der durch das erste Messsystem bestimmten mindestens einen geometrischen Referenz.

### Kurze Beschreibung der Zeichnungen

Nachfolgend wird eine Ausführung der vorliegenden Erfindung anhand eines Beispiels beschrieben. Das Beispiel der Ausführung wird durch die folgenden beigelegten Figuren illustriert:
- Figur 1:: zeigt ein Blockdiagramm, welches schematisch eine ophthalmologische Mess- vorrichtung illustriert, welche ein triangulierendes Messsystem, ein interfero- metrisches Messsystem sowie Verarbeitungsmittel umfasst.
- Figur 2:: zeigt einen Querschnitt durch ein Auge mit verschiedenen geometrischen Struk- turen.
- Figur 3a:: zeigt ein Blockdiagramm, welches schematisch ein triangulierendes Messsystem illustriert, das einen Lichtprojektor und eine Kamera umfasst.
- Figur 3b:: zeigt ein Blockdiagramm, welches schematisch ein triangulierendes Messsystem illustriert, das einen Lichtprojektor und zwei Kameras umfasst
- Figur 4:: zeigt ein Blockdiagramm, welches schematisch ein interferometrisches Messsys- tem illustriert, das ein Interferometer und einen Scanner umfasst.
- Figur 5:: zeigt ein Blockdiagramm, welches schematisch eine Anordnung eines triangulie- renden Messsystems und eines interferometrischen Messsystems der ophthalmo- logischen Messvorrichtung illustriert.
- Figur 6:: zeigt ein Blockdiagramm, welches schematisch eine rotierbare Anordnung eines triangulierenden Messsystems und eines interferometrischen Messsystems der ophthalmologischen Messvorrichtung illustriert.
- Figur 7:: zeigt ein Blockdiagramm, welches schematisch eine weitere rotierbare Anord- nung eines triangulierenden Messsystems und eines interferometrischen Mess- systems der ophthalmologischen Messvorrichtung illustriert.
- Figur 8:: zeigt ein Blockdiagramm, welches schematisch eine Scanebene des interfero- metrischen Messsystems illustriert, die in der Ebene des Lichtspaltes des triangu- lierenden Messsystems liegt.
- Figur 9:: zeigt ein Blockdiagramm, welches schematisch eine weitere Anordnung des interferometrischen Messsystems illustriert, bei der die Scanebene des interfe- rometrischen Messsystems in der Ebene des Lichtspaltes des triangulierenden Messsystems liegt.
- Figur 10:: zeigt ein Querschnittdiagramm, welches schematisch einen im Referenzmessbe- reich des triangulierenden Messsystems beweglichen Detailmessbereich des in- terferometrischen Messsystems illustriert.
- Figur 11:: zeigt ein Querschnittdiagramm, welches schematisch einen im Referenzmessbe- reich des triangulierenden Messsystems fixierten Detailmessbereich des interfe- rometrischen Messsystems illustriert.

### Wege zur Ausführung der Erfindung

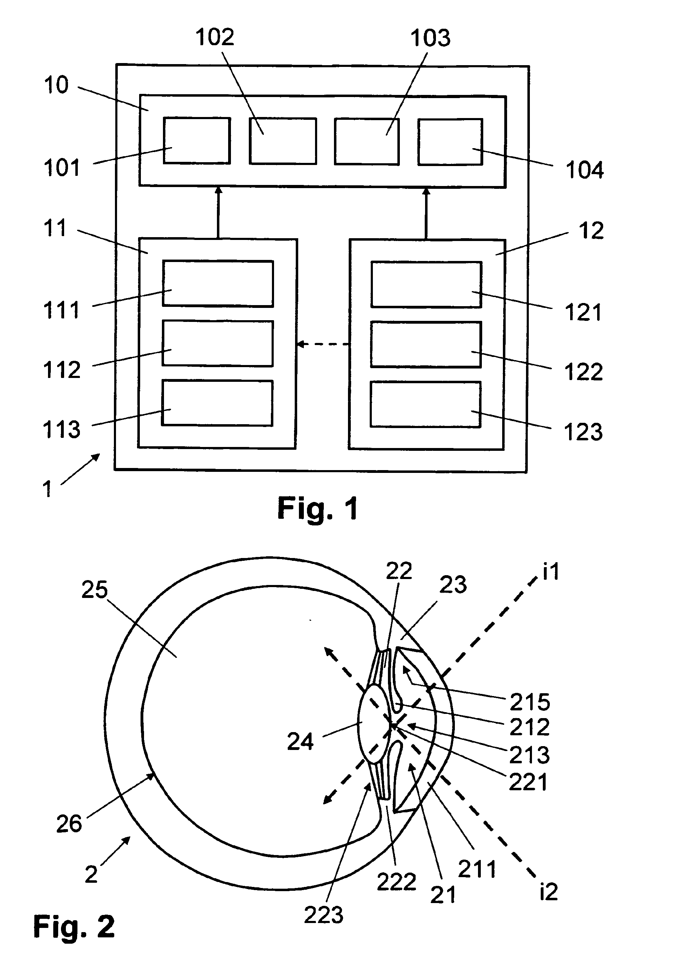

In den Figuren 1 bis 11 bezeichnen gleiche Bezugszeichen einander funktional entsprechende Komponenten, Teile und Systeme; die spezifischen Ausführungen dieser einander entsprechenden funktionalen Komponenten, Teile und Systeme können jedoch in unterschiedlichen Ausführungsvarianten variieren.

In der Figur 1 ist eine ophthalmologische Messvorrichtung 1 zum Bestimmen von geometrischen Strukturen eines Auges 2 dargestellt. Wie in der Figur 2 ersichtlich ist, umfassen diese geometrischen Strukturen beispielsweise Teilstrukturen oder vollständige Strukturen im Vorderkammerbereich 21, wie Cornea 211, Iris 212 und Pupille 213, oder Vorderkammerwinkel 215, im Hinterkammerbereich 22 (respektive daran angrenzend), wie Linsenvorderseite 221, Ziliarkörper 222 oder Zonulafasern 223, im Bereich um die Augenkammer, wie Schlemmscher Kanal 23, und im Bereich hinter der Augenkammer, wie Linse 24, Glaskörper 25 oder Retina 26, sowie Einschlüsse und Implantate, insbesondere transparente Implantate, im Vorder- 21 oder Hinterkammerbereich 22.

Die Messvorrichtung 1 umfasst ein optisches, triangulierendes Messsystem 11 zum Bestimmen von mindestens einer (zwei- oder dreidimensionalen) geometrischen Referenz im Auge 2 durch Triangulation, ein optisches, interferometrisches Messsystem 12 zum Bestimmen von (zwei- oder dreidimensionalen) geometrischen Detailstrukturen im Auge 2 durch optische Interferometrie, und Verarbeitungsmittel 10 zum Positionieren im Auge 2 von den durch das interferometrische Messsystem 12 bestimmten geometrischen Detailstrukturen basierend auf der durch das triangulierende Messsystem 11 bestimmten mindestens einen geometrischen Referenz im Auge 2.

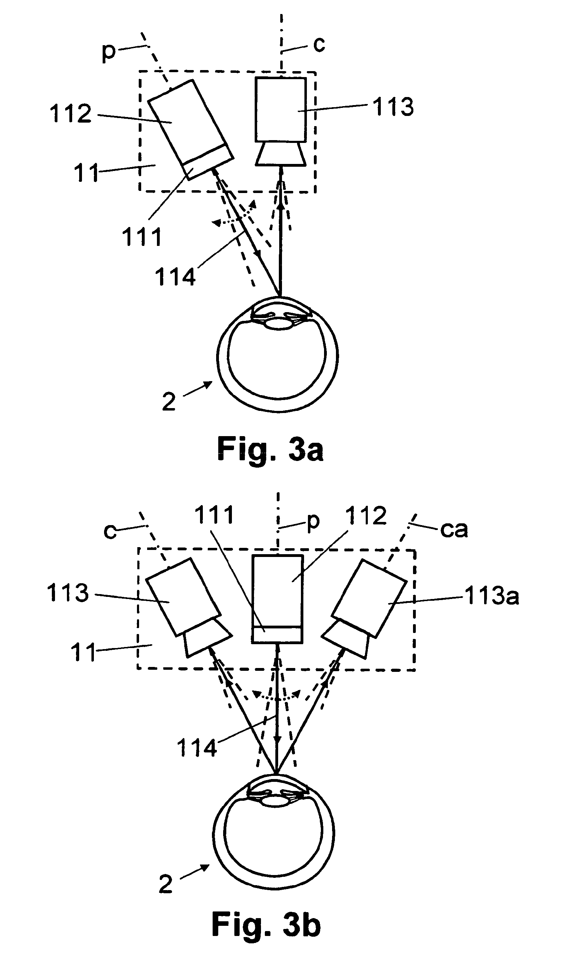

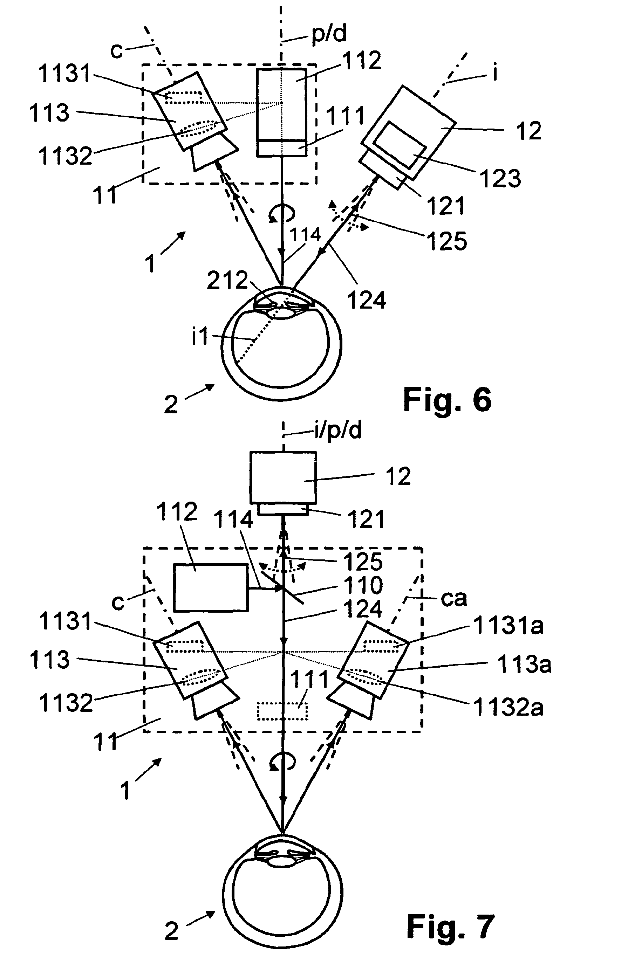

Wie in den Figuren 1, 3a, 3b, 5, 6, 7 schematisch dargestellt ist, umfasst das triangulierende Messsystem 11 einen Lichtprojektor 112 zur Projektion eines Lichtstrahls, vorzugsweise eines Lichtspalts 114, und ein Scanmodul 111 zum Bewegen des Lichtstrahls respektive des Lichtspalts 114 an verschiedene Stellen des Auges 2, sowie eine vorzugsweise in Scheimpflugkonfiguration angeordnete Kamera 113 zum Erfassen von durch den Lichtstrahl erzeugten diffusen Reflexionen am Auge 2 respektive durch den Lichtspalt 114 beleuchteten Querschnittsteilen des Auges 2. Wie in den Figuren 3a, 3b, 5, 6, 7 dargestellt ist, sind der Lichtprojektor 112 und die Kamera 113 im triangulierenden Messsystem 11 so angeordnet, dass die Projektionsachse p des Lichtprojektors 112 und die optische Achse c der Kamera - und dementsprechend ausgestrahlte respektive reflektierte Lichtstrahlen - einen Winkel bilden. Dadurch können Abstandswerte - und damit die Lage und Ausgestaltung - von zwei- und dreidimensionalen geometrischen Strukturen als geometrische Referenzen im Auge 2 durch die Verarbeitungsmittel 10 durch Auswertung von Dreiecksbeziehungen (Triangulation) basierend auf den durch die Kamera 113 erfassten Reflexionen respektive beleuchteten Querschnittsteilen bestimmt werden. Bei der gleichzeitigen Ausstrahlung von mehreren Messstrahlen, insbesondere bei der bevorzugten Projektion eines Lichtspalts 114, kann somit ohne störende Bewegungsartefakte ein instantanes Bild von geometrischen Strukturen im Auge 2 erzeugt werden. In den Figuren 3b, 7 ist eine besonders vorteilhafte Konfiguration des triangulierenden Messsystems 11 dargestellt, mit zwei ausserhalb der Projektionsachse p angeordneten, vorzugsweise in Scheimpflugkonfiguration angeordneten Kameras 113, 113a, deren optische Achsen c, ca vorzugsweise spiegelsymmetrisch zur Projektionsachse p angeordnet sind, zur Erfassung der diffusen Reflexionen am Auge 2 respektive der durch den Lichtspalt 114 beleuchteten Querschnittsteilen des Auges 2 aus zwei Aufnahmeperspektiven. Je nach Ausführungsvariante ist der Scanner 111 eingerichtet die Bewegung des Lichtstrahls respektive des Lichtspalts 114 an verschiedene Stellen des Auges 2 durch Auslenken eines Spiegels und/oder mittels eines Bewegungstreibers durch translatorische oder rotatorische mechanische Bewegung des Lichtprojektors 112 auszuführen. Wie in den Figuren 6 und 7 schematisch angedeutet ist, ist ein Scanner 111 mit einem Rotationstreiber insbesondere in Kombination mit einer einfachen oder doppelt ausgeführten Scheimpflugkamera gemäss der Figur 3b besonders bevorzugt.

Wie in den Figuren 1, 4, 5, 6, 7 schematisch dargestellt ist, umfasst das interferometrische Messsystem 12 ein Interferometer 123 und ein Scanmodul 121 um an mehreren unterschiedlichen Positionen des Auges 2 einen Lichtstrahl 124 vom Interferometer 123 auf und ins Auge 2 zu projizieren und vom Auge 2 reflektiertes Licht zu Erfassen und dem Interferometer 123 zuzuführen (die Projektionsoptik zur fokussierten Projektion des Lichtstrahls 124 ist nicht dargestellt). In einer Variante sind das Interferometer 123 und der Scanner 121 für die bidirektionale Lichtübertragung zwischen dem Interferometer 123 und dem Scanner 121 über ein optisches Übertragungssystem 122 gekoppelt, zB. über einen Lichtfaserleiter. In einer weiteren Variante ist das Interferometer 123 über das optische Übertragungssystem 122 mit dem Lichtprojektor 112 respektive Scanner 111 des triangulierenden Messsystems 11 gekoppelt um von der Lichtquelle des Interferometers 123 das Licht für den Lichtspalt 114 des triangulierenden Messsystems 11 zu liefern.

In einer Ausführungsvariante weisen der vom Lichtprojektor 112 projizierte Lichtstrahl respektive Lichtspalt 114 und der Lichtstrahl 124 vom Interferometer 123 unterschiedliche Wellenlängen auf. Je nach Art des Gewebes kann durch die Wahl der Wellenlänge eine Signalverbesserung erreicht werden. So wird beispielsweise kurzwelliges Licht (< 500nm) besonders stark von der Cornea 211 gestreut. Langwelliges Licht (> 1000nm) eignet sich wegen seiner geringeren Streuung besonders gut für die Penetration des opaken Gewebes des Vorderkammerwinkels 23. Die Wellenlänge des projizierten Lichtspalts 114 weist beispielsweise eine Wellenlänge von weniger als 500nm und der Lichtstrahl 124 vom Interferometer 123 eine Wellenlänge von mehr als 1000nm insbesondere von 1300nm auf.

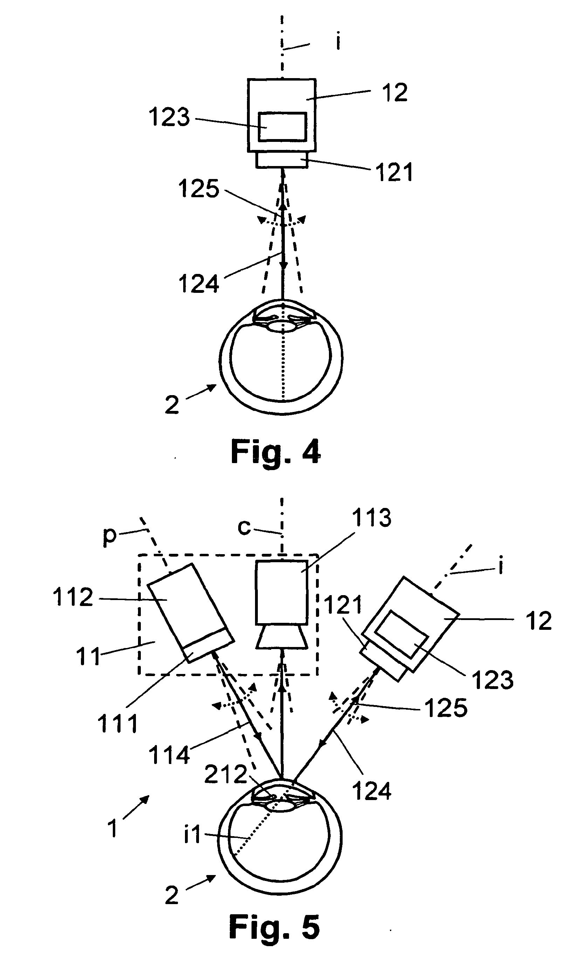

Wie in den Figuren 4, 5, 6, 7 schematisch illustriert ist, strahlt das interferometrische Messsystem 12 einen Lichtstrahl 124 entlang der Projektionsachse i auf und in das Auge 2, und erfasst den insbesondere an den Gewebe- und Materialübergängen vom Augengewebe und Implantaten vielschichtig reflektierten Lichtstrahl 125 entlang dieser Projektionsachse i. Basierend auf Interferenz und somit Laufzeit führt das Interferometer 123 punktuell eine axiale Abstandsmessung - und damit Lagebestimmung - von geometrischen Detailstrukturen mit unterschiedlichem Brechungsindex im Auge durch (A-Scan). Durch Bewegen der Projektionsachse i-und damit der Ausrichtung der projizierten und reflektierten Lichtstrahlen 124, 125 - mittels des Scanners 121 nacheinander an mehrere Augenpositionen, liefert die interferometrische Abstandsmessung Abstandswerte - und damit die Ausgestaltung - von zwei- und dreidimensionalen geometrischen Detailstrukturen im Auge 2. Das Interferometer 123 ist nicht nur in der Lage die Grenzschichten von geometrischen Strukturen zu bestimmen sondern kann dahingehend erweitert werden, dass auch doppelbrechende Strukturen visualisiert werden, spektroskopische Informationen gewonnen werden, und Fluidströmungen innerhalb des Gewebe sichtbar gemacht werden können. Wie in der Figur 2 schematisch dargestellt ist, ermöglicht die interferometrische axiale Distanzbestimmung entlang geeignet ausgerichteter Projektionsachsen i1. i2, die Vermessung von Bereichen im Auge 2, beispielsweise den Vorderkammerwinkel 215 im Vorderkammerbereich 21 oder Bereiche hinter der Iris 212 im Hinterkammerbereich 22, die für die bekannten triangulierenden Verfahren optisch nicht einsehbar sind. Je nach Ausführungsvariante ist der Scanner 121 eingerichtet die Bewegung der Projektionsachse i und/oder der Ausrichtung der projizierten und reflektierten Lichtstrahlen 124, 125 durch Auslenken eines Spiegels und/oder mittels eines Bewegungstreibers durch translatorische oder rotatorische mechanische Bewegung des für die Projektion des Lichtstrahls 124 vom interferometrischen Messsystem 12 verwendeten Lichtprojektors auszuführen.

Die Verarbeitungsmittel 10 umfassen einen oder mehrere Prozessoren sowie Programm- und Datenspeicher, in welchem verschiedene programmierte Softwaremodule zur Steuerung des Prozessors gespeichert sind. Die Softwaremodule umfassen beispielsweise ein Triangulationsmodul 101, ein Interferometriemodul 102, ein Positionierungsmodul 103 und ein Kompilierungsmodul 104.

Das Triangulationsmodul 101 ist eingerichtet, aus den durch die Kamera 113 erfassten und gespeicherten diffusen Reflexionen respektive durch den Lichtspalt 114 beleuchteten Querschnittsteilen des Auges 2 durch Triangulationsberechnungen innerhalb eines definierten Referenzmessbereichs die Lage und Ausgestaltung von geometrischen Referenzen (zB. die oben angeführten zwei- und dreidimensionalen geometrischen (Teil-) Strukturen) im Auge 2 zu bestimmen.

Das Interferometriemodul 102 ist eingerichtet, basierend auf mehreren erfassten und gespeicherten axialen interferometrischen Abstandsmessungen (AScans) des Interferometers 123 innerhalb eines definierten Detailmessbereichs die Ausgestaltung von geometrischen Detailstrukturen im Auge 2 zu bestimmen (B-Scan, C-Scan).

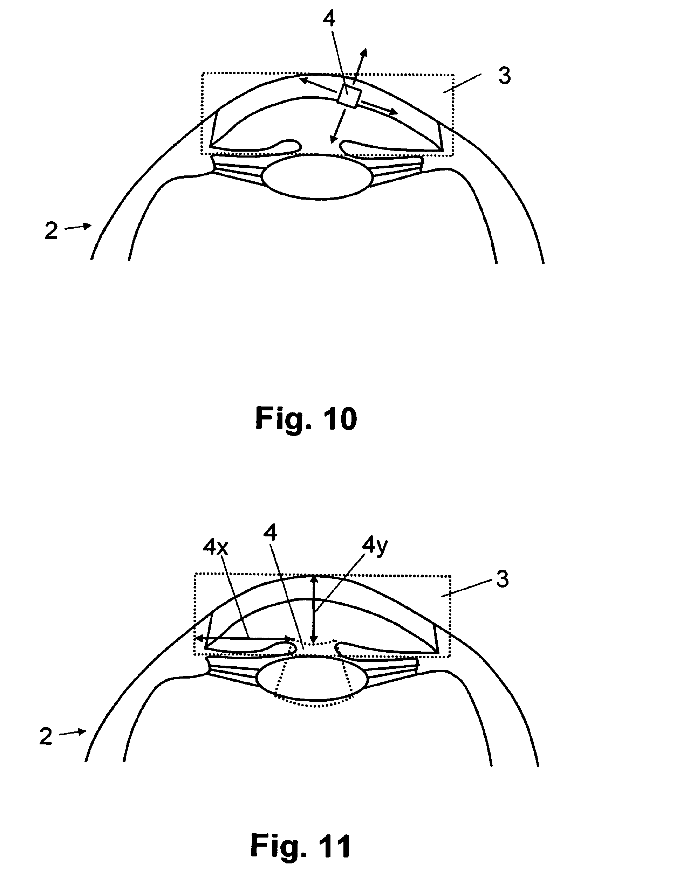

Das Positionierungsmodul 103 ist eingerichtet, eines oder mehrere definierte geometrische Referenzmerkmale des Auges 2 zu bestimmen, und die geometrischen Detailstrukturen basierend auf diesen geometrischen Referenzmerkmalen im Auge 2 zu positionieren. Die geometrischen Referenzmerkmale umfassen beispielsweise die oben angeführten Strukturen, Teilstrukturen, Einschlüsse, Implantate und/oder darauf erkennbare (zweidimensionale) Formen, Flecken, Markierungen oder Muster, die vom Positionierungsmodul 103, beispielsweise mittels Bildverarbeitungsfunktionen, einerseits in den geometrischen Referenzen innerhalb des triangulierend bestimmten Referenzmessbereichs 3 und andererseits in den interferometrisch bestimmten geometrischen Detailstrukturen innerhalb des Detailmessbereichs 4 bestimmt werden (siehe Figur 10), so dass auf der Basis der geometrischen Referenzmerkmale die Positionierung des Detailmessbereichs 4 im Referenzmessbereich 3, respektive die räumliche Lage der Detailstrukturen im Auge 2 ermittelt werden kann.

In einer Ausführungsvariante ist das Positionierungsmodul 103 zudem eingerichtet, auf der Basis der durch das triangulierende Messsystem 11 bestimmten Lage und Ausgestaltung der dreidimensionalen geometrischen Strukturen im Auge 2, insbesondere auf der Basis der durch diese Strukturen bestimmten Grenzflächen zwischen Gewebe- und Materialübergängen von Augengewebe und Implantaten, sowie auf der Basis von Brechungsindizes der verschiedenen Augengewebe- und Implantatbereichen den erwarteten Strahlverlauf des Lichtstrahls 124 vom Interferometer 123 durch das Auge 2 zu bestimmen und diesen erwarteten Strahlverlauf zusätzlich zur Positionierung des Detailmessbereichs 3 im Referenzmessbereich 4 zu verwenden.

In einer weiteren Ausführungsvariante ist das Positionierungsmodul 103 überdies eingerichtet, vom Benutzer Instruktionen entgegenzunehmen, beispielsweise über eine grafische/visuelle Benutzerschnittstelle, zur Bestimmung und Selektion einer oder mehrerer geometrischen Zielreferenzen im Auge (2) in Bezug zu den bestimmten, beispielsweise visualisierten, zweidimensionalen oder dreidimensionalen geometrischen Strukturen im Auge 2. Das Positionierungsmodul 103 leitet dann die geometrischen Zielreferenzen, beispielsweise als Koordinaten oder Vektoren, an das interferometrische Messsystem 12, weiches dann die Projektionsachse i und somit den Lichtstrahl 124 vom Interferometer 123 zur interferometrischen Erfassung des Detailmessbereichs 4 auf diese Zielreferenz ausrichtet. Dies Funktion ist insbesondere sinnvoll zum wieder finden von sich pathologisch verändernden Gewebebereiche über längere Zeiträume.

Wie in den Figuren 10 und 11 schematisch dargestellt ist, ist das interferometrische Messsystem 12 eingerichtet, die geometrischen Detailstrukturen in einem zwei- oder dreidimensionalen Detailmessbereich 4 des Auges 2 zu bestimmen, der im Verhältnis zum zwei- oder dreidimensionalen Referenzmessbereich 3, der vom triangulierenden Messsystem 11 vermessen wird, wesentlich kleiner dimensioniert ist und innerhalb des Referenzmessbereichs 3 liegt, den Referenzmessbereich 3 überlappt, an den Referenzmessbereich 3 angrenzt oder völlig ausserhalb des Referenzmessbereichs 3 angeordnet ist.

In der Variante nach Figur 10 ist das interferometrische Messsystem 12 respektive dessen projizierter Lichtstrahl 124 während der Durchführung der Messung bezüglich des triangulierenden Messsystems 11, und damit der Detailmessbereich 4 bezüglich des Referenzmessbereichs 3, frei verschiebbar, so dass die geometrischen Detailstrukturen in mehreren Detailmessbereichen 4 bestimmt und über geometrische Referenzmerkmale bezüglich des Referenzmessbereichs 3 positioniert werden können. Vorteilhaft lassen sich somit, kompaktere, kostengünstigere Projektionsoptiken realisieren. Weiterhin können so auch interferometrische (OCT-) Techniken eingesetzt werden, die einen begrenzten Tiefenmessbereich haben (z.B. Frequency Domain OCT), bzw. mit hoher optische Auflösung betrieben werden sollen.

In der Variante nach Figur 11 wird das interferometrische Messsystem 12 vor der Durchführung der Messung über geometrische Einstellwerte 4x, 4y, die beispielsweise einer über einen Kalibrierungskörper definierten geometrischen Referenz entsprechen, bezüglich des triangulierenden Messsystems 11 mechanisch fixiert (gekoppelt), so dass die räumliche Lage des Detailmessbereichs 4, und damit die Lage der geometrischen Detailstrukturen, bezüglich des Referenzmessbereichs 3 fest definiert ist. Die definierte geometrische Referenz auf die das triangulierende Messsystem 11 und das interferometrische Messsystem 12 zur Kalibrierung eingestellt werden, ist beispielsweise die Pupille 213, die Linsenvorderseite 221 und/oder der Scheitelpunkt der Cornea 211, welche vorzugsweise über einen Kalibrierkörper oder aber durch ein konkretes Auge 2 repräsentiert werden.

Das Kompilierungsmodul 104 ist eingerichtet, mehrere im Auge 2 verschieden positionierte geometrische Detailstrukturen zu erfassen und zu speichern, diese geometrischen Detailstrukturen respektive die zugehörigen Detailmessbereiche 4 basierend auf den ermittelten geometrischen Referenzen/Referenzmerkmalen im Referenzmessbereich 3 und somit gegenseitig zu positionieren und zu einer übergeordneten kombinierten geometrischen Detailstruktur zusammenzusetzen. Das Kompilierungsmodul 104 ist überdies eingerichtet, die einzelnen geometrischen Detailstrukturen nach deren Positionierung im Referenzmessbereich 3 durch Strecken, Stauchen und/oder Verzerren so zu verformen, dass sie in ihren aneinandergrenzenden oder überlappenden Übergangsbereichen möglichst kontinuierlich zusammenpassen.

In den folgenden Abschnitten werden mit Bezug zu den Figuren 5, 6, 7, 8 und 9 verschiedene Ausführungen der Messvorrichtung 1 mit unterschiedlichen Anordnungen und Kombinationen des triangulierenden Messsystems 11 und des interferometrischen Messsystems 12 beschrieben.

In der Ausführung nach Figur 5 verläuft die optische Achse c der Kamera 113 im Wesentlichen entlang der Sehachse des Auges 2 und die schräg zur optischen Achse c der Kamera 113 verlaufende Ausrichtung der Projektionsachse p des Lichtprojektors 112 wird vom Scanner 111 zum Abtasten des Auges 2 mittels eines beweglichen Ablenkspiegels verändert Die Projektionsachse i des interferometrischen Messsystems 12 ist schräg zur optischen Achse c der Kamera 113 ausgerichtet, so dass triangulatorisch nicht erfassbare Bereiche, beispielsweise Bereiche hinter der Iris 212, vermessen werden können, wie mit der Projektionsachse i1 schematisch angezeigt wird. Der Scanner 121 ermöglicht durch Verschieben und/oder Auslenken der Projektionsachse i in der Zeichenebene und/oder senkrecht zur Zeichenebene das Auge 2 über einen ausgedehnten Detailmessbereich 4 interferometrisch zu vermessen.

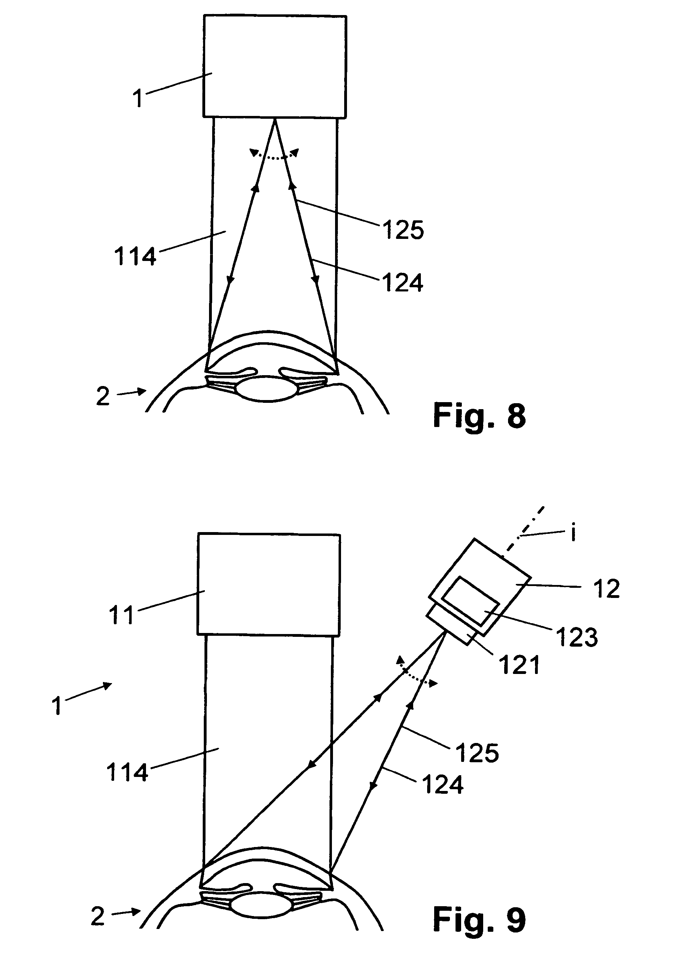

In der Ausführung nach Figur 6 verläuft die Projektionsachse p des Lichtprojektors 112 im Wesentlichen entlang der Sehachse des Auges 2 und die optische Achse c der Kamera 113 ist schräg zur Projektionsachse p des Lichtprojektors 112 ausgerichtet und in Scheimpflugkonfiguration angeordnet, d.h. die Linsenebene der Kameralinse 1132 und die Bilderfassungsebene des Bildaufzeichners 1131 schneiden sich in einem gemeinsamen Punkt auf der Projektionsachse p des Lichtprojektors 112. Die Kamera 113 und der Lichtprojektor 112 sind miteinander mechanisch fixiert und werden von einem Bewegungstreiber des Scanners 111 um eine durch die Projektionsachse p verlaufende Drehachse d rotiert, was eine vollumfängliche Abtastung des Auges 2 mit dem Lichtstrahl respektive Lichtspalt 114 ermöglicht. Die Projektionsachse i des interferometrischen Messsystems 12 ist schräg zur Projektionsachse p des Lichtprojektors 112 ausgerichtet, so dass triangulatorisch nicht erfassbare Bereiche, beispielsweise Bereiche hinter der Iris 212, vermessen werden können, wie mit der Projektionsachse i1 schematisch angezeigt wird. Der Scanner 121 ermöglicht durch Verschieben und/oder Auslenken der Projektionsachse i in der Zeichenebene und/oder senkrecht zur Zeichenebene das Auge 2 über einen ausgedehnten Detailmessbereich 4 interferometrisch zu Vermessen. In einer bevorzugten Variante ist das interferometrische Messsystem 12, oder zumindest der Scanner 121, mit dem Lichtprojektor 112 (und der Kamera 113) mechanisch fixiert und wird vom Bewegungstreiber um die Drehachse d mitrotiert. Vorzugsweise ist der Scanner 121 in dieser Variante so eingerichtet, dass die Projektionsachse i und somit der projizierte Lichtstrahl 124 in einer Scanebene ausgelenkt respektive bewegt wird, die in der Ebene des Lichtspalts 114 verläuft, wie in der Figur 9 dargestellt ist.

In der Ausführung nach Figur 7 verlaufen die Projektionsachse p des Lichtprojektors 112 und die (zentrale) Projektionsachse i des interferometrischen Messsystems 12 in einer gemeinsamen Achse, die beispielsweise entlang der Sehachse des Auges 2 verläuft. Zu diesem Zweck werden der projizierte Lichtstrahl respektive Lichtspalt 114 des Lichtprojektors 112 und/oder der projizierte Lichtstrahl 124 des Interferometers 123 über einen oder mehrere Umlenkspiegel 110 auf diese gemeinsame Achse eingespiegelt. Um den reflektierten Lichtstrahl 125 dem Interferometer 123 zuzuführen ist der Spiegel 110 halbdurchlässig ausgestaltet. Die Kamera 113 ist wie im Zusammenhang mit Figur 6 beschrieben in Scheimpflugkonfiguration zum Lichtprojektor 112 angeordnet und mit dem Lichtprojektor 112 mechanisch fixiert. Optional weist die Messvorrichtung 1 zudem eine zweite in Scheimpflugkonfiguration angeordnete Kamera 113a auf, deren Kameralinse 1132a und Bildaufzeichner 1131 a mit der Kameralinse 1132 und dem Bildaufzeichner 1131 spiegelsymmetrisch zur Projektionsachse p des Lichtprojektors 112 angeordnet sind. Der Lichtprojektor 112 und die Kamera(s) 113, 113a und das interferometrische Messsystem 12, oder zumindest dessen Scanner 121, werden vom Bewegungstreiber des Scanners 111 um eine ebenfalls durch die Projektionsachse p verlaufende Drehachse d rotiert. Vorzugsweise ist der Scanner 121 in dieser Variante so eingerichtet, dass die Projektionsachse i und somit der projizierte Lichtstrahl 124 in einer Scanebene ausgelenkt respektive bewegt wird, die in der Ebene des Lichtspalts 114 verläuft, wie in der Figur 8 dargestellt ist.

In einer kombinierten Variante der Figuren 6 und 7, ist das interferometrische Messsystem 12 so eingerichtet, dass seine Projektionsachse i einerseits für interferometrische Vermessungen in triangulatorisch nicht erfassbaren Bereichen, beispielsweise hinter der Iris 212, in eine schräg zur Projektionsachse p des Lichtprojektors 112 verlaufende Ausrichtung gebracht werden kann, und andererseits für möglichst homogene triangulierende und interferometrische Messbereiche, in eine mit der Projektionsachse p des Lichtprojektors 112 zusammenfallende Ausrichtung gebracht werden kann.

## Patentansprüche

1. Ophthalmologische Messvorrichtung (1) zum Bestimmen von geometrischen Strukturen eines Auges (2), umfassend ein optisches, triangulierendes erstes Messsystem (11) und ein optisches, interferometrisches zweites Messsystem (12), wobei das erste Messsystem (11) eingerichtet ist zur Bestimmung von mindestens einer geometrischen Referenz im Auge (2) durch Triangulation, **dadurch gekennzeichnet,**
**dass** das zweite Messsystem (12) eingerichtet ist zur Bestimmung von geometrischen Detailstrukturen im Auge (2) durch optische Interferometrie, und
**dass** die Messvorrichtung (1) eingerichtet ist, die durch das zweite Messsystem (12) bestimmten geometrischen Detailstrukturen basierend auf der durch das erste Messsystem (11) bestimmten mindestens einen geometrischen Referenz im Auge (2) zu positionieren.

2. Messvorrichtung (1) nach Anspruch 1, **dadurch gekennzeichnet, dass** das erste Messsystem (11) und das zweite Messsystem (12) mechanisch so gekoppelt sind, dass die geometrischen Detailstrukturen im Auge (2) bei einer der geometrischen Referenz entsprechenden Stellung des ersten Messsystems (11) basierend auf einem oder mehreren geometrischen Einstellwerten positionierbar sind.

3. Messvorrichtung (1) nach einem der Ansprüche 1 oder 2, **dadurch gekennzeichnet, dass** die Messvorrichtung (1) ein Positionierungsmodul (103) umfasst, welches eingerichtet ist, eines oder mehrere geometrische Referenzmerkmale des Auges (2) zu bestimmen, und die geometrischen Detailstrukturen basierend auf den geometrischen Referenzmerkmalen im Auge (2) zu positionieren.

4. Messvorrichtung (1) nach einem der Ansprüche 1 bis 3, **dadurch gekennzeichnet, dass** das erste Messsystem (11) einen Lichtprojektor (112) zur Projektion eines Lichtspalts (114) und ein erstes Scanmodul (111) zum Bewegen des Lichtspalts an verschiedene Stellen des Auges (2) umfasst, und dass das zweite Messsystem (12) ein Interferometer (123) und ein zweites Scanmodul (121) umfasst, wobei das zweite Scanmodul (121) eingerichtet ist, an mehreren unterschiedlichen Positionen des Auges (2) mindestens einen Lichtstrahl (124) vom Interferometer (123) auf und ins Auge (2) zu projizieren und vom Auge (2) reflektiertes Licht dem Interferometer (123) zuzuführen.

5. Messvorrichtung (1) nach Anspruch 4, **dadurch gekennzeichnet, dass** das erste Scanmodul (111) eingerichtet ist, den Lichtspalt (114) entlang einer Scanrichtung zu bewegen, und dass das zweite Scanmodul (121) eingerichtet ist, den Lichtstrahl (124) in einer zur Scanrichtung im Wesentlichen parallel oder im Wesentlichen normal verlaufenden Scanebene zu bewegen.

6. Messvorrichtung (1) nach Anspruch 4, **dadurch gekennzeichnet, dass** das erste Scanmodul (111) eingerichtet ist, den Lichtspalt (114) durch Rotation um eine im Wesentlichen im Lichtspalt (114) verlaufende Drehachse (d) zu bewegen, und dass das zweite Scanmodul (121) eingerichtet ist, den Lichtstrahl (124) in einer Scanebene zu bewegen, wobei eine zentrale Projektionsachse des zweiten Scanmoduls (121) mit der Drehachse (d) einen Winkel bildet oder entlang der Drehachse (d) verläuft.

7. Messvorrichtung (1) nach einem der Ansprüche 5 oder 6, **dadurch gekennzeichnet, dass** das erste Scanmodul (111) und das zweite Scanmodul (121) so eingerichtet und gekoppelt sind, dass der Lichtspalt (114) und die Scanebene in einer gemeinsamen Ebene liegen.

8. Messvorrichtung (1) nach einem der Ansprüche 4 bis 7, **dadurch gekennzeichnet, dass** der vom Lichtprojektor (112) projizierte Lichtspalt (114) und der Lichtstrahl (124) vom Interferometer (123) unterschiedliche Wellenlängen aufweisen, beispielsweise beträgt die Wellenlänge des projizierten Lichtspalts (114) weniger als 500nm und die Wellenlänge des Lichtstrahls (124) vom Interferometer (123) mehr als 1000nm insbesondere 1300nm.

9. Messvorrichtung (1) nach einem der Ansprüche 4 bis 8, **dadurch gekennzeichnet, dass** das erste Messsystem (11) mindestens eine in Scheimpflugkonfiguration angeordnete Kamera (113) zum Erfassen von durch den Lichtspalt (114) beleuchteten Querschnittsteilen des Auges (2) umfasst.

10. Messvorrichtung (1) nach einem der Ansprüche 1 bis 9, **dadurch gekennzeichnet, dass** die Messvorrichtung (1) ein Kompilierungsmodul (104) umfasst, welches eingerichtet ist, mehrere im Auge (2) verschieden positionierte geometrische Detailstrukturen zu erfassen, diese geometrischen Detailstrukturen basierend auf der mindestens einen geometrischen Referenz gegenseitig zu positionieren und zu einer übergeordneten kombinierten geometrischen Detailstruktur zusammenzusetzen.

11. Messvorrichtung (1) nach Anspruch 10, **dadurch gekennzeichnet, dass** das Kompilierungsmodul (104) eingerichtet ist, die geometrischen Detailstrukturen zum Zusammensetzen der übergeordneten kombinierten geometrischen Detailstruktur passend zu verformen.

12. Messvorrichtung (1) nach einem der Ansprüche 1 bis 11, **dadurch gekennzeichnet, dass** das erste Messsystem (11) eingerichtet ist, die geometrische Referenz in einem definierten Referenzmessbereich (3) des Auges (2) zu bestimmen, dass das zweite Messsystem (12) eingerichtet ist, die geometrischen Detailstrukturen in einem im Verhältnis zum definierten Referenzmessbereich (3) wesentlich kleineren Detailmessbereich (4) des Auges (2) zu bestimmen, und dass die Messvorrichtung (1) Bewegungstreiber zum Bewegen des zweiten Messsystems (12) umfasst zum Bestimmen von geometrischen Detailstrukturen in mehreren verschiedenen Detailmessbereichen des Auges.

13. Messvorrichtung (1) nach Anspruch 12, **dadurch gekennzeichnet, dass** der Detailmessbereich (4) des Auges (2) in Bezug zum definierten Referenzmessbereich (3) gemäss mindesten einer aus den nachfolgenden Optionen angeordnet werden kann: innerhalb des definierten Referenzmessbereichs (3), überlappend mit dem definierten Referenzmessbereich (3), angrenzend an den definierten Referenzmessbereich (3) und ausserhalb des definierten Referenzmessbereichs (3).

14. Messvorrichtung (1) nach einem der Ansprüche 1 bis 13, **dadurch gekennzeichnet, dass** das erste Messsystem (11) und das zweite Messsystem (12) eine gemeinsame Lichtquelle aufweisen.

15. Ophthalmologisches Messverfahren zum Bestimmen von geometrischen Strukturen eines Auges (2), umfassend:
Bestimmen von mindestens einer geometrischen Referenz im Auge (2) durch ein optisches, triangulierendes erstes Messsystem (11),
Bestimmen von geometrischen Detailstrukturen im Auge (2) durch ein optisches interferometrisches zweites Messsystem (12), und
Positionieren der durch das zweite Messsystem (12) bestimmten geometrischen Detailstrukturen im Auge (2) basierend auf der durch das erste Messsystem (11) bestimmten mindestens einen geometrischen Referenz.

## Claims

1. Ophthalmological measuring device (1) for determining geometric structures of an eye (2), comprising an optical, triangulating first measurement system (11) and an optical, interferometric second measurement system (12), with the first measurement system (11) being designed to determine at least one geometric reference in the eye (2) by triangulation, **characterized in that**
the second measurement system (12) is designed to determine geometric detailed structures in the eye (2) by optical interferometry, and
the measuring device (1) is designed to position the geometric detailed structures determined by the second measurement system (12) on the basis of the at least one geometric reference in the eye (2) determined by the first measurement system (11).

2. Measuring device (1) according to Claim 1, **characterized in that** the first measurement system (11) and the second measurement system (12) are mechanically coupled such that the geometric detailed structures in the eye (2) can be positioned on the basis of one or more geometric settings in a position of the first measurement system (11) corresponding to the geometric reference.

3. Measuring device (1) according to either Claim 1 or 2, **characterized in that** the measuring device (1) comprises a positioning module (103) designed to determine one or more geometric reference features of the eye (2) and to position the geometric detailed structures on the basis of the geometric reference features in the eye (2).

4. Measuring device (1) according to one of Claims 1 to 3, **characterized in that** the first measurement system (11) comprises a light projector (112) for projecting a light slit (114) and a first scan module (111) for moving the light slit to different positions of the eye (2), and **characterized in that** the second measurement system (12) comprises an interferometer (123) and a second scan module (121), with the second scan module (121) being designed to project at least one light beam (124) from the interferometer (123) onto and into the eye (2) at a plurality of different positions of the eye (2) and to supply light reflected by the eye (2) to the interferometer (123).

5. Measuring device (1) according to Claim 4, **characterized in that** the first scan module (111) is designed to move the light slit (114) along a scan direction, and **characterized in that** the second scan module (121) is designed to move the light beam (124) in a scan plane substantially parallel to or substantially normal to the scan direction.

6. Measuring device (1) according to Claim 4, **characterized in that** the first scan module (111) is designed to move the light slit (114) by rotation about a rotational axis (d) substantially running in the light slit (114), and **characterized in that** the second scan module (121) is designed to move the light beam (124) in a scan plane, with a central projection axis of the second scan module (121) forming an angle with the rotational axis (d) or running along the rotational axis (d).

7. Measuring device (1) according to either Claim 5 or 6, **characterized in that** the first scan module (111) and the second scan module (121) are designed and coupled such that the light slit (114) and the scan plane are situated in a common plane.

8. Measuring device (1) according to one of Claims 4 to 7, **characterized in that** the light slit (114) projected by the light projector (112) and the light beam (124) from the interferometer (123) have different wavelengths; for example, the wavelength of the projected light slit (114) is less than 500 nm and the wavelength of the light beam (124) from the interferometer is more than 1000 nm, more particularly 1300 nm.

9. Measuring device (1) according to one of Claims 4 to 8, **characterized in that** the first measurement system (11) comprises at least one camera (113) arranged in Scheimpflug configuration for registering cross-sectional parts of the eye (2) illuminated by the light slit ( 114).

10. Measuring device (1) according to one of Claims 1 to 9, **characterized in that** the measuring device (1) comprises a compilation module (104) designed to register a plurality of geometric detailed structures positioned differently in the eye (2), to position these geometric detailed structures with respect to one another on the basis of the at least one geometric reference and to assemble said structures to form a superordinate combined geometric detailed structure.

11. Measuring device (1) according to Claim 10, **characterized in that** the compilation module (104) is designed to deform suitably the geometric detailed structures for assembling the superordinate combined geometric detailed structure.

12. Measuring device (1) according to one of Claims 1 to 11, **characterized in that** the first measurement system (11) is designed to determine the geometric reference in a defined reference measurement region (3) of the eye (2), that the second measurement system (12) is designed to determine the geometric detailed structures in a detailed measurement region (4) of the eye (2), which detailed measurement region is significantly smaller than the defined reference measurement region (3), and that the measuring device (1) comprises movement drivers for moving the second measurement system (12) for determining geometric detailed structures in a plurality of different detailed measurement regions of the eye.

13. Measuring device (1) according to Claim 12, **characterized in that** the detailed measurement region (4) of the eye (2) can be arranged with respect to the defined reference measurement region (3) as per at least one of the following options: within the defined reference measurement region (3), overlapping with the defined reference measurement region (3), adjoining the defined reference measurement region (3) and outside of the defined reference measurement region (3).

14. Measuring device (1) according to one of Claims 1 to 13, **characterized in that** the first measurement system (11) and the second measurement system (12) have a common light source.

15. Ophthalmological measurement method for determining geometric structures of an eye (2), comprising:
determining at least one geometric reference in the eye (2) by an optical, triangulating first measurement system (11),
determining geometric detailed structures in the eye (2) by an optical interferometric second measurement system (12), and
positioning the geometric detailed structures in the eye (2) determined by the second measurement system (12) on the basis of the at least one geometric reference determined by the first measurement system (11).

## Revendications

1. Dispositif de mesure ophtalmologique (1) pour déterminer des structures géométriques d'un oeil (2), comprenant un premier système de mesure optique de triangulation (11) et un deuxième système de mesure optique interférométrique (12), le premier système de mesure (11) étant installé pour la détermination par triangulation d'au moins une référence géométrique dans l'oeil (2), **caractérisé en ce que**
le deuxième système de mesure (12) est installé pour la détermination par interférométrie optique de structures géométriques de détails dans l'oeil (2), et **en ce que**
le dispositif de mesure (1) est installé pour positionner les structures géométriques des détails déterminées par le deuxième système de mesure (12) en se basant sur au moins une référence géométrique dans l'oeil (2) déterminée par le premier système de mesure (11).

2. Dispositif de mesure (1) selon la revendication 1, **caractérisé en ce que** le premier système de mesure (11) et le deuxième système de mesure (12) sont accouplés mécaniquement de manière à pouvoir positionner les structures géométriques des détails dans l'oeil (2) dans une position de premier système de mesure (11) correspondant à la référence géométrique en se basant sur une ou plusieurs valeurs de réglage géométrique.

3. Dispositif de mesure (1) selon une des revendications 1 ou 2, **caractérisé en ce que** le dispositif de mesure (1) comporte un module de positionnement (103) installé pour déterminer une ou plusieurs marques de référence géométrique de l'oeil (2), et pour positionner les structures géométriques des détails en se basant sur les marques de référence géométrique de l'oeil (2).

4. Dispositif de mesure (1) selon une des revendications 1 à 3, **caractérisé en ce que** le premier système de mesure (11) comporte un projecteur de lumière (112) pour projeter d'une fente lumineuse (114) et un premier module de balayage (111) pour déplacer la fente lumineuse vers différents emplacements de l'oeil (2), et **en ce que** le deuxième système de mesure (12) comporte un interféromètre (123) et un deuxième module de balayage (121), le deuxième module de balayage (121) étant installé pour projeter, en plusieurs positions différentes de l'oeil (2), au moins un faisceau lumineux (124) de l'interféromètre (123) sur et dans l'oeil (2) et pour renvoyer la lumière réfléchie par l'oeil (2) vers l'interféromètre (123).

5. Dispositif de mesure (1) selon la revendication 4, **caractérisé en ce que** le premier module de balayage (111) est installé pour déplacer la fente lumineuse (114) le long d'une direction de balayage, et **en ce que** le deuxième module de balayage (121) est installé pour déplacer le faisceau lumineux (124) dans un plan de balayage pour l'essentiel parallèle ou pour l'essentiel normal à la direction de balayage.

6. Dispositif de mesure (1) selon la revendication 4, **caractérisé en ce que** le premier module de balayage (111) est installé pour déplacer la fente lumineuse (114) par rotation autour d'un axe de rotation (d) se propageant pour l'essentiel dans la fente lumineuse (114), et **en ce que** le deuxième module de balayage (121) est installé pour déplacer le faisceau lumineux (124) dans un plan de balayage dans lequel un axe de projection central du deuxième module de balayage (121) forme un angle avec l'axe de rotation (d) ou bien se propage le long de l'axe de rotation (d).

7. Dispositif de mesure (1) selon une des revendications 5 ou 6, **caractérisé en ce que** le premier module de balayage (111) et le deuxième module de balayage (121) sont installés et accouplés de telle sorte que la fente lumineuse (114) et le plan de balayage se trouvent dans un plan commun.

8. Dispositif de mesure (1) selon une des revendications 4 à 7, **caractérisé en ce que** la fente lumineuse (114) projetée par le projecteur de lumière (112) et le faisceau lumineux (124) de l'interféromètre (123) présentent des longueurs d'ondes différentes, par exemple la longueur d'onde de la fente lumineuse (114) projetée se monte à moins de 500 nm et la longueur d'onde du faisceau lumineux (124) provenant de l'interféromètre (123) se monte à plus de 1000 nm, notamment 1300 nm.

9. Dispositif de mesure (1) selon une des revendications 4 à 8, **caractérisé en ce que** le premier système de mesure (11) comporte au moins une caméra (113) disposée dans la configuration de Scheimpflug pour enregistrer des parties de l'oeil (2) en coupe transversale éclairées par la fente lumineuse (114).

10. Dispositif de mesure (1) selon une des revendications 1 à 9, **caractérisé en ce que** le dispositif de mesure (1) comporte un module de compilation (104) installé pour enregistrer plusieurs structures géométriques de détails positionnées de différentes manières dans l'oeil (2), pour positionner mutuellement ces structures géométriques de détails basées sur au moins une référence géométrique et pour les assembler en une structure géométrique de détails combinée d'ordre supérieur.

11. Dispositif de mesure (1) selon la revendication 10, **caractérisé en ce que** le module de compilation (104) est installé pour assembler les structures géométriques de détails et pour déformer de manière appropriée la structure géométrique de détails combinée d'ordre supérieur.

12. Dispositif de mesure (1) selon une des revendications 1 à 11, **caractérisé en ce que** le premier système de mesure (11) est installé pour déterminer la référence géométrique dans un espace défini de mesure de référence (3) de l'oeil (2), **en ce que** le deuxième système de mesure (12) est installé pour déterminer les structures géométriques de détails dans un espace de mesure de détails (4) de l'oeil (2) sensiblement plus petit par rapport à l'espace défini de mesure de référence (3), et **en ce que** le dispositif de mesure (1) comporte des actionneurs de mouvement pour déplacer le deuxième système de mesure (12) pour la détermination de structures géométriques de détails dans plusieurs espaces de mesure de détails de l'oeil.

13. Dispositif de mesure (1) selon la revendication 12, **caractérisé en ce que** l'espace de mesure de détails (4) de l'oeil (2) peut être arrangé par rapport à l'espace défini de mesure de référence (3) selon au moins une des options suivantes : à l'intérieur de l'espace défini de mesure de référence (3), en chevauchement avec l'espace défini de mesure de référence (3), en jouxtant l'espace défini de mesure de référence (3) et à l'extérieur de l'espace défini de mesure de référence (3).

14. Dispositif de mesure (1) selon une des revendications 1 à 13, **caractérisé en ce que** le premier système de mesure (11) et le deuxième système de mesure (12) comportent une source lumineuse commune.

15. Procédé de mesure ophtalmologique pour déterminer des structures géométriques d'un oeil (2), comprenant :
la détermination d'au moins une référence géométrique dans l'oeil (2) au moyen d'un premier système de mesure optique (11) par triangulation,
la détermination de structures géométriques de détails dans l'oeil (2) au moyen d'un deuxième système de mesure (12) par interférométrie optique, et
le positionnement des structures géométriques des détails dans l'oeil (2) déterminées par le deuxième système de mesure (12) en se basant sur au moins une référence géométrique déterminée par le premier système de mesure (11).
